(19) Europäisches Patentamt
European Patent Office
Office européen des brevets

(11) Veröffentlichungsnummer: **0 410 280 A1**

# (12) EUROPÄISCHE PATENTANMELDUNG

(21) Anmeldenummer: **90113720.8**

(22) Anmeldetag: **18.07.90**

(51) Int. Cl.[5]: **C07C 217/08**, C07C 213/00, G01N 33/531

(30) Priorität: **26.07.89 DE 3924705**

(43) Veröffentlichungstag der Anmeldung: **30.01.91 Patentblatt 91/05**

(84) Benannte Vertragsstaaten: **AT BE CH DE DK ES FR GB GR IT LI LU NL SE**

(71) Anmelder: **BOEHRINGER MANNHEIM GMBH**
**Sandhofer Strasse 116**
**D-6800 Mannheim 31(DE)**

(72) Erfinder: **Herrmann, Rupert, Dr. rer. nat.**
**In der Au 23**
**D-8120 Weilheim(DE)**
Erfinder: **Huber, Erasmus, Dr. rer. nat.**
**St. Willibald 10**
**D-8911 Unterfinning(DE)**
Erfinder: **Josel, Hans-Peter, Dr. rer. nat.**
**Eisvogelstrasse 26**
**D-8120 Weilheim(DE)**
Erfinder: **Klein, Christian, Dr. rer. nat.**
**Blütenstrasse 16**
**D-8120 Weilheim(DE)**
Erfinder: **Zink, Bruno**
**Seeblickstrasse 4**
**D-8114 Uffing(DE)**

## (54) Heterobifunktionelle Verbindungen.

(57) Gegenstand der Anmeldung sind Verbindungen der Formel Ia

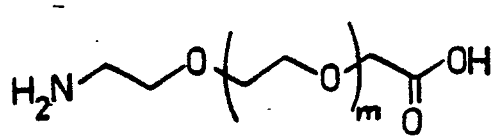

(Ia)

in denen m eine Zahl von 1 bis 10 ist sowie deren Salze und deren Verwendung als Linkermoleküle.

EP 0 410 280 A1

## HETEROBIFUNKTIONELLE VERBINDUNGEN

Gegenstand der Erfindung sind spezielle heterobifunktionelle Verbindungen zur Verknüpfung von aminogruppenhaltigen Verbindungen mit carbonsäurehaltigen Verbindungen, Verfahren zu ihrer Herstellung und mit ihrer Hilfe hergestellte Konjugate.

Konjugate von immunologisch aktiven Verbindungen, wie beispielsweise Haptenen, mit anderen Verbindungen, beispielsweise Nukleinsäuren, sind bekannt. Sie werden in jüngerer Zeit vermehrt als Bestandteile von Reagenzien zur immunologischen Bestimmung bestimmter Analyten in der Humandiagnostik eingesetzt.

Als Verbindungen, mit denen eine Verknüpfung von Haptenen mit anderen Verbindungen vorgenommen wird sind homo- und heterobifunktionelle Verbindungen bekannt. Die homobifunktionellen Verbindungen, wie sie beispielsweise aus der EP-A-0254172 oder der US-A-4760142 bekannt sind, haben den Nachteil, daß es beispielsweise schwierig ist, selektiv Konjugate aus Haptenen und Proteinen herzustellen. Es entstehen als Produkt auch Konjugate aus zwei Haptenen bzw. zwei Proteinen. Heterobifunktionelle Verbindungen als Verknüpfungsreagenz vermeiden diese Nachteile. Aus der DE-A-2631656 sind Hydroxysuccinimidesterderivate der Propansäure bekannt, die auf einer Seite an ein Protein, wie Nierenacylase, und an der anderen Seite ebenfalls mit einem Protein oder aber mit einem festen Träger verbunden werden können. Eine selektive und schonende Bindung von aminogruppenhaltigen Verbindungen an carboxylgruppenhaltige Verbindungen ist mit diesen Verbindungen nicht möglich.

In der EP-A-0042626 bzw. der US-A-4372974 ist die Verwendung von 2-Aminoethoxyessigsäure als Arzneimittel beschrieben. Die Herstellung dieser Verbindung gelang nach bekannten Methoden. Die Herstellung höherer Homologen der Aminoethoxyessigsäure nach dieser Methode hat sich als nicht möglich erwiesen.

In der EP-A-0102118 ist die Herstellung eines Gemisches von Acylaminoethoxyessigsäuren beschrieben. Diese Verbindungen werden als Zusatzstoff in Badezusätzen verwendet. Sie sind als Verknüpfungsreagenzien nicht geeignet, da sie keine freie Aminogruppe aufweisen und zudem die Verwendung eines Gemisches den hohen Anforderungen an eine spezifische Verknüpfung nicht gerecht wird.

Es bestand daher ein Bedarf an der Bereitstellung von Verbindungen, mit denen selektiv carboxylgruppenhaltige Verbindungen mit aminogruppenhaltigen Verbindungen über eine hydrophile Brücke definierter Länge verknüpft werden können.

Diese Aufgabe wird gelöst durch die Bereitstellung von Verbindungen der Formel I

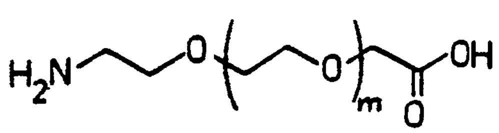

(Ia)

in der m eine natürliche Zahl von 1 bis 10 ist.

Ebenfalls Gegenstand der Erfindung sind ein besonders günstiges Verfahren zur Herstellung von Verbindungen der Formel Ib

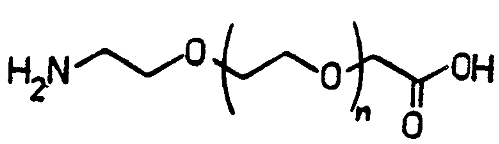

(Ib)

in der n eine natürliche Zahl von 0 bis 10 ist, die Verwendung der Verbindungen zur Herstellung von Konjugaten aus carboxylgruppenhaltigen und aminogruppenhaltigen Verbindungen, sowie diese Konjugate selbst.

Die Herstellung der erfindungsgemäßen Verbindungen der Formel Ib gelang durch eine neue besonders vorteilhafte Synthese. Ein Gegenstand der Erfindung ist daher ein Verfahren zur Herstellung von Verbindungen der Formel Ib durch Umsetzung von Verbindungen der Formel II

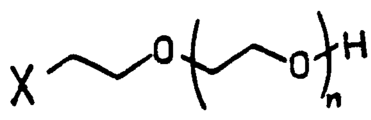

(II)

in der X ein Phthalimidrest oder Halogen, bevorzugt Chlor, Brom oder Jod ist und n wie bei Formel Ib definiert ist,
mit einem Diazoessigsäurederivat der Formel III
$N_2CH$-COOR (III)
in der R ein Alkylrest mit 1-6 Kohlenstoffatomen, bevorzugt ein Ethylrest, ist, unter Metallsalzkatalyse, bevorzugt Kupfer-, Palladium- oder Rhodiumsalzen, besonders bevorzugt Rhodium-II-salzkatalyse, umgesetzt wird, gegebenenfalls, wenn X ein Halogen ist, Umsetzung des entstandenen Produkts mit einem Reagenz zur Einführung einer Aminogruppe, bevorzugt Phthalimid-Kalium, unter nukleophilen Substitutionsbedingungen und anschließender Abspaltung gegebenenfalls vorhandener Schutzgruppen bzw. Hydrolyse des Esters, bevorzugt in HC1.

Alle Reaktionsschritte finden bevorzugt in Lösung statt. Bevorzugt werden die Verbindungen der Formel I - III vor ihrer Weiterverarbeitung isoliert.

Ebenfalls Gegenstand der Erfindung sind die Salze, auch innere Salze, der Verbindungen der allgemeinen Formel Ib. Sie können schon bei der Herstellung der Verbindungen, oder aber nachträglich durch Umsetzen mit allgemein üblichen Säuren oder Basen entstehen. Bevorzugt sind die Alkali-Salze der Carboxylatgruppe und die Halogenide des Ammoniumrests.

Durch das erfindungsgemäße Verfahren ist es möglich, Homologe der Aminoethoxyessigsäure herzustellen, die eine vorherbestimmte Anzahl von Ethylenoxy-Einheiten aufweisen. Dies ist besonders wichtig, wenn die hergestellten Verbindungen der Formel Ib zur Verknüpfung von Haptenen mit Proteinen oder Nukleinsäuren verwendet werden sollen. Die Länge de Kette zwischen dem Amino-und dem Carboxylende ist kritisch für die weitere Verwendung der Konjugate. Die Größe von n richtet sich auch nach der Größe des Proteins, mit welchem das Hapten verknüpft werden soll. Besonders bevorzugt ist n = 1 bis 6. So ist beispielsweise die Zugänglichkeit des Haptens für einen Antikörper gegen dieses Hapten etwas eingeschränkt, wenn n = O ist (2-Aminoethoxyessigsäure).

Zur Herstellung von Konjugaten aus einer carboxylgruppenhaltigen Verbindung und einer aminogruppenhaltigen Verbindung mit Hilfe von Verbindungen der Formel Ib gibt es mehrere Möglichkeiten, die sich in der Reihenfolge der Umsetzung der Komponenten miteinander unterscheiden. Bevorzugt ist folgendes Vorgehen:

- Zunächst wird die Verbindung der Formel Ib mit einer Verbindung, deren Carboxylgruppe beispielsweise durch Bildung eines reaktiven Esters, Halogenids oder Anhydrids für nukleophile Substitutionen aktiviert wurde, umgesetzt. Solche aktivierten Carboxylgruppen sind bekannt. Als besonders bevorzugt haben sich mit N-Hydroxysuccinimid veresterte Carboxylgruppen erwiesen.
- Durch die Reaktion der Verbindung der Formel Ib mit der carboxylgruppenhaltigen Verbindung entsteht eine Verbindung der Formel IV

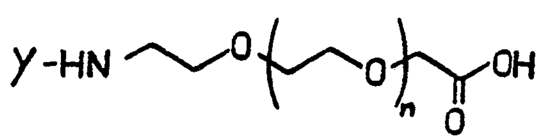

(IV)

in der Y der Rest einer carboxylgruppenhaltigen Verbindung ist und n wie oben definiert ist. Die Bindung erfolgt über die Carboxylgruppe.

- Diese Verbindung der Formel IV kann nun mit aminogruppenhaltigen Verbindungen zu einer Verbindung der Formel V

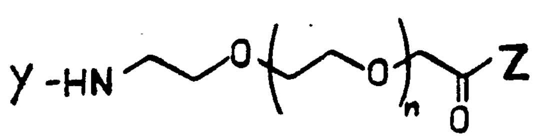

(V)

in der Y und n wie oben definiert sind und Z der Rest einer aminogruppenhaltigen Verbindung ist, umgesetzt werden.

Dies kann mit Hilfe von Kupplungsreagenzien geschehen oder nach vorheriger Überführung der Verbindung der Formel (IV) in ein aktiviertes Derivat. Zur Aktivierung können dieselben Methoden verwendet werden, wie oben für die carboxylgruppenhaltige Verbindung beschrieben.

Die Carboxylgruppe der Verbindung der Formel (IV) wird dazu bevorzugt in einen aktivierten Ester, Anhydrid etc. überführt. Anschließend wird mit der amingruppenhaltigen Verbindung nach bekannten Methoden umgesetzt.

Das Verfahren zur Verknüpfung von aminogruppenhaltigen und carboxylgruppenhaltigen Verbindungen laßt sich auch in umgekehrter Reihenfolge durchführen, falls z.B. die Aminogruppe zuerst in geschützter Form vorliegt.

Neben dem stufenweisen Aufbau dieser durch heterobifunktionellen Verbindungen verknüpften Konjugaten, können auch direkt aktivierte Derivate der Verbindung I eingesetzt werden. Dazu werden analog zum Stand der Technik reaktive Gruppen eingeführt. Z.B. ann die Aminogruppe in ein Isocyanat, ein Isothiocyanat oder ein Maleimid umgewandelt werden, bzw. Acrylsäurereste, Halogenacetylreste oder Maleimidoalkansäurereste eingeführt werden. Besonders bevorzugt ist die Umwandlung/Einführung in eine Maleimidfunktion bzw. eines Iodacetylrestes. Die Carboxylgruppe kann durch Umsetzung z.B. zu einem Ester, Halogenid oder Anhydrid aktiviert werden, besonders bevorzugt ist ein N-Hydroxysuccinimidester oder ein p-Nitrophenylester. Die Verbindungen gemäß der Formel VI

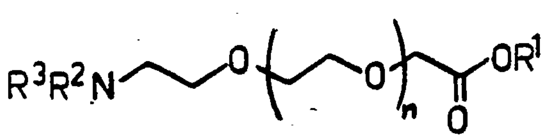

(VI)

wobei $-NR^2R^3$ eine aktivierte Aminogruppe, $COOR^1$ eine aktivierte Carboxylgruppe und n wie oben definiert ist, eignen sich hervorragend zur direkten Konjugatsynthese z.B. von Protein-Protein-Konjugaten, besonders bevorzugt sind Antikörper-Enzym-Konjugate. Z.B. lassen sich, falls die Verbindung der Formel VI einen Maleimidorest und eine N-Hydroxysuccinimidestergruppe enthält, zuerst analog zum Stand der Technik aminogruppenhaltige Proteine ankuppeln und im zweiten Schritt SH-gruppenhaltige Proteine.

Beispiele für Verbindungen, die über entsprechende Amino- oder Carbonsäure-Gruppen verfügen, sind Haptene, Biotin, Farbstoffe, insbesondere Fluoreszenzfarbstoffe, Nukleinsäuren, Proteine, z.B. auch (Strept-)Avidin und Antikörper, Enzyme, Glycokonjugate, Peptide, etc.

Bevorzugt sind die primäre Aminogruppen enthaltenden aminogruppenhaltigen Verbindungen. Bevorzugte Verbindungen sind Nukleinsäuren und Proteine. Die Proteine können anhand ihrer Funktion in verschiedene Klassen eingeteilt werden: Enzyme, Strukturproteine, Antikörper etc. Besonders bevorzugt ist die Verknüpfung mit alkalischer Phosphatase, β-Galaktosidase oder Peroxidase.

Bevorzugte carboxylgruppenhaltige Verbindungen sind Haptene und Fluoreszenzfarbstoffe. Besonders bevorzugt sind Haptene. Unter Haptenen werden sämtliche Verbindungen verstanden, die als solche nicht in der Lage sind, in vivo eine Immunantwort zu induzieren; bei Verknüpfung mit einem geeigneten Trägermolekül ist es jedoch möglich, Antikörper gegen sie zu erzeugen. Dazu gehören beispielsweise Biotin, Digoxigenin, Cortisol-3-carboxymethyloxim etc. Diese Haptene haben bevorzugt keine zusätzlichen ungeschützten stark nukleophilen Reste, die mit den aktivierten Carboxylgruppen reagieren können. Als Beispiel für ein Hapten mit einem geschützten nukleophilen Rest kann N-BOC-Thyroxin genannt werden. Ein Fluoreszenzfarbstoff mit aktivierter Carboxylgruppe ist beispielsweise Carboxyfluorescein-N-hydroxysuccinimid-ester.

Das erfindungsgemäße Verfahren ist besonders gut zur Verknüpfung von Haptenen mit Proteinen über die jeweiligen Amino- bzw. Carboxylgruppen geeignet.

Bei Verwendung von Verbindungen der Formel Ib ist es möglich, Konjugate der Formel V herzustellen, bei denen die biologische Funktion der aminogruppenhaltigen verbindung, z.B. Enzymaktivität, Bindefähigkeit zu einem entsprechenden Immunpartner oder Hybridisierungsfähigkeit weitgehend erhalten bleibt.

Andererseits hat es sich überraschenderweise herausgestellt, daß die Affinität eines Antikörpers zum freien Hapten genauso hoch oder nur unwesentlich geringer ist, als die zu einem mit Hapten und Linker gemäß Formel I hergestellten Immunogen. Die Linker aus Formel I zeigen demgemäß keine Immunogenizität. Auch die Reaktion des Haptens mit einem biospezifischen Bindungspartner, wie im Fall von Biotin mit Avidin oder Streptavidin, wird nicht unmöglich gemacht.

Die Verbindungen der Formeln Ib und IV sowie deren aktivierte Derivate haben den Vorteil, daß sie in polaren Lösungsmitteln insbesondere in wässrigen Lösungen gut löslich sind. Dies begünstigt die Kopplung an wasserlösliche Verbindungen, beispielsweise Proteine. Auch die Verbindungen der Formel V sind in wässrigen Lösungen gut löslich. Dies ist besonders wichtig für die Durchführung von Immunoassays oder Hybridisierungstests direkt in physiologischen Proben. Beispielsweise haben sich Brückenglieder mit mehr als 2 benachbarten Methylengruppen als zu hydrophob erwiesen.

Die Bindungen in den erfindungsgemäßen Konjugaten sind aber auch ausreichend fest, da es sich bei den Bindungen der Brücke mit dem Hapten bzw. dem Protein um kovalente Bindungen, insbesondere Amidbindungen handelt. Daher hat sich die Verknüpfung zweier Arten von Verbindungen über eine Aminogruppe und eine Carboxylgruppe als besonders vorteilhaft erwiesen.

Die vorliegende Erfindung wird durch die folgenden Beispiele weiter erläutert:

## Beispiel 1

Synthese von 2(2(2-Aminoethoxy-)ethoxy-)ethoxy-essigsäurehydrochlorid I, n = 2.

a) Zu 84.25 g Triethylenglycol-mono-chlorid in 400 ml Tetrahydrofuran (THF) werden 105 ml Diazoessigester, gelöst in 150 ml THF innerhalb von 3 h zugestropft. Im Abstand von 30 Min. werden jeweils 50 mg Rhodium-II-acetat hinzugefügt. Anschließend wird mit Methanol versetzt, im Vakuum eingedampft und im Hochvak. destilliert.

Kp (0.13 mbar) = 120-125° C; Ausbeute = 45,5 g

b) 12.7 g des in a) hergestellten Produktes werden in 100 ml DMF gelöst und mit 9.25 g Kalium-Phthalimid und einer katalytischen Menge 18-Krone-6 versetzt. Man rührt 2,5 h bei 100° C, entfernt das DMF im Hochvakuum, nimmt in Essigester auf, filtriert und wäscht zweimal mit $H_2O$. Nach Trocknen und Eindampfen erhält man einen öligen Rückstand.

Ausbeute: 16 g IV n = 2

NMR($CDCl_3$/TMS ppm):

1.21-1.35 $-CH_3$;

3.59-4.31 $-CH_2-$

7.66-7.90 Phthalyl

c) Synthese von I (n=2):

3.6 g IV (n=2) werden in 100 ml 20% HCl unter Rückfluß gerührt, nach 1.5 h im Vakuum eingeengt, der Rückstand in 100 ml $H_2O$ aufgenommen und abfiltriert. Das Filtrat wird dreimal mit Essigester extrahiert und eingeengt. Das Produkt wird über Sephadex/$H_2O$ gereinigt.

Ausbeute: 1.1 g I (n=2)

NMR(DMSO-d6/TMS ppm):

3.57 $-CH_2-CH_2-$

4.03 $-CH_2-$

4.14 $NH_3^+$

Eine weitere Aufreinigung kann durch Überführung in das BOC-geschützte Produkt mit anschließender Säulenchromatographie und Abspaltung erfolgen.

## Beispiel 2

Umsetzung von I (n=1) mit Biotin

370 mg I (n=1) werden zusammen mit 775 mg Biotin-N-hydroxysuccinimidester und 3mmol Triethylamin in 10 ml DMF gelöst und 24 h bei Raumtemperatur gerührt. Das Lösungsmittel wird entfernt, der Rückstand in Aceton aufgenommen, abfiltriert und erneut eingeengt. Man erhält ca. 800 mg Rohprodukt, welches durch Chromatographie an Kieselgel weiter aufgereinigt wird. (Eluens: Chloroform: Methanol: Eisessig 9:1:0.1)

Rf = 0.2 (CMA 9:3:0.5)

## Beispiel 3

Umsetzung von I (n = 2) mit Digoxigenin-3-carboxymethylether-N-hydroxysuccinimidester.

0.36 g I (n = 2) werden in 10 ml abs. DMF gelöst und mit 0.6 ml Triethylamin vesetzt. Anschließend werden 0.54 g Digoxigenin-3-carboxymethylether-N-hydroxysuccinimidester hinzugefügt und 16 h bei Raumtemperatur gerührt. Das Lösungsmittel wird entfernt und der Rückstand zweimal über eine Kieselgelsäure (Eluens Methanol: Tetrahydrofuran 1:1 + 0.5% Essigsäure) gereinigt.
Ausbeute: 0.38 g Rf = 0.58 (Essigester : Essigsäure 9:1)

Beispiel 4

Synthese von 2(2-Aminoethoxy-)ethoxy-essigsäure-hydrochlorid I, n = 1.

a) Zu 62.25 g Diethylenglycol-mono-chlorid in 150 ml Tetrahydrofuran (THF) werden 105 ml Diazoessigester, gelöst in 150 ml THF innerhalb von 3 h zugestropft. Im Abstand von 30 Min. werden jeweils 50 mg Rhodium-II-acetat hinzugefügt. Anschließend wird mit Methanol versetzt, im Vakuum eingedampft und im Hochvak. destilliert.
Kp (0.1 mbar) = 80-88° C; Ausbeute = 45,6 g
b) 21.07 g des in a) hergestellten Produktes werden in 200 ml DMF gelöst und mit 18.5 g Kalium-Phthalimid und einer katalytischer Menge 18-Krone-6 versetzt. Man rührt 2,5 h bei 100° C, entfernt das DMF im Hochvakuum, nimmt in Essigester auf, filtriert und wäscht zweimal mit $H_2O$. Nach Trocknen und Eindampfen erhält mal einen öligen Rückstand.
NMR($CDCl_3$/TMS ppm):
1.21-1.35 $-CH_3$;
3.59-4.31 $-CH_2-$
7.66-7.90 Phthalyl
Rf (Ethylacetat/Petrolether 2/1) = 0.53
c) Synthese von I (n = 1):
25.24 g IV (n = 1) werden in 500 ml 20% HCl unter Rückfluß gerührt, nach 1.5 h im Vakuum eingeengt, der Rückstand in 100 ml $H_2O$ aufgenommen und abfiltriert. Das Filtrat wird dreimal mit Essigester extrahiert und eingeengt. Das Produkt wird über Sephadex/$H_2O$ gereinigt.
Ausbeute: 9.37 g I (n = 1)
NMR(DMSO-d6/TMS ppm):
3.57 $-CH_2-CH_2-$
4.03 $-CH_2-$
4.14 $NH_3+$
Rf (Methylethylketon/AcOH/$H_2O$ 3/1/1) = 0.56
Eine weitere Aufreinigung kann durch Überführung in das BOC-geschützte Produkt mit anschließender Säulenchromatographie und Abspaltung erfolgen.

Beispiel 5

Umsetzung von I (n = 2) mit Biotin

1.6 g I (n = 2) werden zusammen mit 642 mg Biotin-N-hydroxysuccinimidester und 1.5 ml Triethylamin in 30 ml DMF gelöst und 2 h bei Raumtemperatur gerührt. Das Lösungsmittel wird entfernt, der Rückstand in Aceton aufgenommen, abfiltriert und erneut eingeengt.
Rf (Eisessig/Ethylacetat/Wasser 5/5/2) = 0.53

**Ansprüche**

1) Verbindungen der allgemeinen Formel Ia

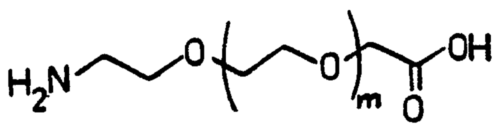
(Ia)

wobei m eine natürliche Zahl von 1 bis 10 ist und ihre Salze.

2) Verbindungen der allgemeinen Formel VI

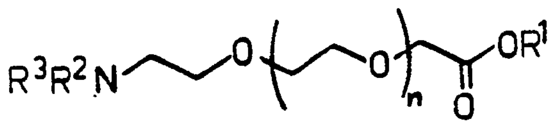
(VI)

in der

$R^1$ Wasserstoff, ein p-Nitrophenylrest, ein N-Succinimidrest, ein Halogenidrest oder ein $C_1$-$C_6$ Alkylcarbonylrest,

$R^2$ und $R^3$, die gleiche oder verschieden sein können, Wasserstoff, eine Halogenacetylgruppe, ein Acrylsäurerest oder ein Maleimidoalkansäurerest

oder die Gruppe $-NR^2R^3$ eine Maleimidogruppe, ein Isocyanat-oder ein Isothiocyanatrest ist,

und wobei $R^2$ under $R^3$ nicht gleichzeitig Wasserstoff bedeuten, wenn $R^1$ Wasserstoff ist,

und in der

n eine natürliche Zahl von 0 bis 10 ist.

3) Verfahren zur Herstellung von Verbindungen der Formel Ib

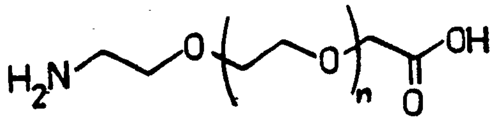
(Ib)

in der n eine natürliche Zahl von 0 bis 10 ist, dadurch gekennzeichnet, daß eine Verbindung der Formel II

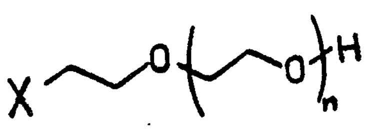
(II)

in der n wie oben definiert ist und

X ein Halogen oder ein Phthalimidrest ist

mit einer Verbindung der Formel III

$N_2CH-COOR$ (III)

in der R ein Alkylrest mit 1-6 Kohlenstoffatomen bedeutet umgesetzt wird und für den Fall, daß X Halogen bedeutet, das Produkt mit einem Reagenz zur Einführung einer Aminogruppe umgesetzt wird und die Esterfunktion sowie eventuell vorhandene Aminoschutzgruppen hydrolysiert werden.

4) Verwendung der Verbindungen der Formel Ib

$N_2CH-COOR$ (III)

in der n eine natürliche Zahl von 0 bis 10 ist oder ihrer Salze, zur Verknüpfung von Carboxylgruppen aufweisenden Verbindungen mit aminogruppenhaltigen Verbindungen.

5) Verbindungen der Formel V

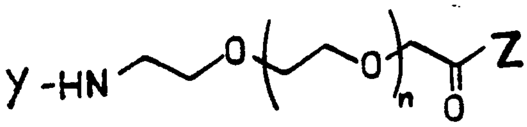

(V)

in der
Y der Rest eines carboxylgruppenhaltigen Haptens, Farbstoffs, Peptids oder Proteins, oder einer Nukleinsäure oder Biotin,
Z der Rest eines aminogruppenhaltigen Haptens, Farbstoffs, Proteins, Peptids oder einer Nukleinsäure und n eine natürliche Zahl von 0 bis 10 ist.
6) Verbindungen der Formel IV

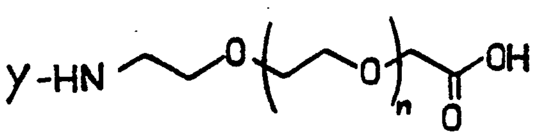

(IV)

in der
Y der Rest eines carboxylgruppenhaltigen Haptens, Farbstoffs, Peptids oder Proteins, oder einer Nukleinsäure oder Biotin und n eine natürliche Zahl von 0 bis 10 ist.

Europäisches Patentamt

# EUROPÄISCHER RECHERCHENBERICHT

Nummer der Anmeldung

EP 90113720.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| X | CHEMICAL ABSTRACTS, Band 93, Nr. 19, 10. November 1980, Columbus, Ohio, USA J.SLAMA et al. "Lectin-mediated aggregation of liposomes containing glycolipids with variable hydrophilic spacer arms" Seite 229, Spalte 1, Zusammenfassung-Nr. 181 439p & Biochemistry 1980,19(20), 4595-600 -- | 1,4 | C 07 C 217/08<br>C 07 C 213/00<br>G 01 N 33/531 |
| X | CHEMICAL ABSTRACTS, Band 94, Nr. 25, 22. Juni 1981, Columbus, Ohio, USA J.SLAMA et al. "The synthesis of alycolipids containing a hydrophilic spacer-group" Seite 632, Spalte 2, Zusammenfassung-Nr. 209 135h & Carbohydr. Res. 1981,88(2), 213-21 -- | 1,4 | |
| | | | RECHERCHIERTE SACHGEBIETE (Int Cl⁵) |
| A | DE - A1 - 2 521 523 (SYVA CO.) * Beispiel 1.5; Ansprüche 2,3 * -- | 5,6 | C 07 C 217/00<br>C 07 C 213/00<br>G 01 N 33/00<br>A 61 K |
| A | US - A - 4 680 338 (SUNDORO) * Ansprüche 1,11,13,15,16,21; Beispiele 3,7; Zusammenfassung * -- | 1,6 | |
| D,A | EP - B1 - 0 042 626 (NEW YORK UNIVERSITY) * Beispiel IV * ---- | 3 | |

Der vorliegende Recherchenbericht wurde für alle Patentansprüche erstellt.

| Recherchenort | Abschlußdatum der Recherche | Prüfer |
|---|---|---|
| WIEN | 11-10-1990 | KÖRBER |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument